# EUROPEAN PATENT SPECIFICATION

(11) **EP 1 910 738 B1**
(45) Date of publication and mention of the grant of the patent: **20.01.2010**
(21) Application number: 05778732.7
(22) Date of filing: 29.07.2005
(51) Int. Cl.: F21V 33/00, A61H 33/00, A47K 3/00, A61N 5/06

(54) **LIGHTING SYSTEM FOR TUBS DESIGNED TO PROVIDE WELL-BEING WITH RELAXATION TECHNIQUES**
ZUR ERHÖHUNG DES WOHLBEFINDENS MIT ENTSPANNUNGSTECHNIKEN AUSGELEGTES BELEUCHTUNGSSYSTEM FÜR WANNEN
SYSTEME D'ECLAIRAGE POUR BAIGNOIRES CONCU POUR PERMETTRE LE BIEN-ETRE GRACE A DES TECHNIQUES DE RELAXATION

(43) Date of publication of application: 16.04.2008
(73) Proprietor: Technodesign SRL, 37020 Negrar (IT)
(72) Inventor: SALGARELLI, Silvio, I-37020 Negrar (VR) (IT)
(74) Representative: Petraz, Gilberto Luigi
(86) International application number: PCT/IT2005/000453
(87) International publication number: WO 2007/013110

(56) References cited:
- EP-A- 1 038 484
- EP-A- 1 075 815
- DE-A1- 4 400 071
- DE-U1-202004 018 797

## Description

### TECHNICAL FIELD

This invention concerns a lighting system for tubs designed for therapeutic and/or beauty and/or dermatological treatments or to provide well-being with relaxation techniques.

This lighting system comprises a series of light fittings with variable chromatics which increase the comfort and well-being of the person during the treatment cycle.

The invention in question proposes the use of particular light fittings which can preferably although not necessarily be used together with a transparent wrapping sheet with a high degree of thermal conductivity for beauty and/or well-being treatments designed for body care.

The lighting system according to the invention allows one to make the most of the energy of colours and light for complete psychological-physical well-being, since it uses particular sources of light which develop their chromatic energy in tubs for relaxation and well-being.

The invention in question can be applied in the beauty treatment and well-being sector and in particular in the sector of dry floatation tubs for physical well-being and beauty treatments.

### BACKGROUND ART

The use of tubs for the application of cosmetic and beauty treatments and for relaxation cycles is known.

These tubs, also known as floatation tubs, are provided with an accessory, one of which is the subject of a patent filed in the name of the applicant hereto, consisting of a particular transparent sheet placed above the body containing the liquid, which can be heated by a specific heating unit with temperature control.

For these types of treatment the body of traditional dry flotation tubs is completely covered by this particular waterproof sheet, in close contact with the heated liquid so as to be able to transmit the heat both by conduction and by other convection means to the person lying on the sheet, without however providing any support to the body, and at the same time sealing the water inside the container.

The sheet is made from transparent, flexible, elastic plastic material so as to easily adapt to the shape of a user who lies down on it and is wrapped in it.

These tubs are particularly suitable for dermatological treatments using mud, seaweed, etc., demonstrating considerable flexibility of use in the beauty treatment sector.

Also known to background art in the beauty treatment and well-being centre sector is the use of light sources which can be fitted in traditional tubs for beauty treatments but more commonly the whirlpool type, which provide the tub with a certain chromatic effect.

In some cases, as in the solution described in the American patent US23172721, there is just one light source installed in such a way that a single light projector directs its light beam inside the tub.

To use this solution it is necessary to make a hole in the body of the tub before fitting the projector and then seal the hole to prevent water leakage.

The limitation of this solution concerns the fact that the light source is punctiform, and the luminous effect cannot therefore be diffused, with a very limited chromatic effect and light distribution.

According to other solutions, such as for example the one proposed in the European patent EP01093783, the light source consists of a plurality of lights distributed over the entire inner surface of the tub. This case, however, presents certain installation problems, since a series of holes have to be made in the body of the tub, each being designed to accommodate a single light source, and in particular water seal problems since although each hole in the tub is sealed and siliconed they represent a potential danger from the point of view of infiltrations and water leakage, which can occur above all with the passage of time or as a result of deterioration caused by the water. DE 20 2004 01 8797 U1, which is considered to represent the most relevant state of the art, discloses a lighting system for tubs designed for therapeutic and/or beauty and/or dermatological treatments or designed to provide well-being with relaxation techniques, comprising a plurality of electronic cards each comprising lighting elements with variable chromatics. Furthermore, the lighting elements are distributed over the inner surface of the tub, fitted to a series of holes that have to be made in the body of the tub, each hole being designed to accommodate a single (group of) light element(s).

### DESCRIPTION OF THE INVENTION

This invention concerns a tub for personal treatments designed to provide well-being with relaxation techniques that can eliminate or significantly reduce the problems described above relative to the light sources commonly used in tubs, employing a new type of lighting system.

This invention proposes in particular to provide a new type of lighting system that can be used in any type of tub for physical well-being and beauty treatments.

This is achieved by means of a lighting system that can be fitted in bath tubs or in particular tubs for beauty treatments and/or for carrying out personal well-being techniques, with the features described in the main claim.

The dependent claims describe advantageous embodiments of the invention.

The lighting system according to the invention comprises rigid tubular pipes in transparent material, equipped with means for fixing them inside the body of a tub for physical well-being and beauty treatments; these rigid pipes, being in direct communication with the outside of the tub, can be fitted internally with a plurality of electronic cards each comprising lighting elements with variable chromatics.

According to the invention these electronic cards, consisting of printed circuits provided with lighting elements formed by LEDs, can be positioned adjacent to each other inside the transparent tubes, using rapid-connection terminal boards which make them easy and quick to change when, for example, the elements are faulty or damaged.

For example, each printed circuit can comprise twelve groups of three different colour LEDs, indicatively red, green and blue.

The luminous tubular elements remain immersed in the water in the tub and, according to a particularly advantageous embodiment of the invention, can be used in conjunction with transparent flotation sheets for tubs used for beauty treatments or relaxation techniques.

When the user lies down in the tub, his/her body is wrapped in the sheet in a floating condition without coming into contact with the water, and this situation activates the lighting system in question which makes it possible to complete the treatment, since the diffused variable chromatic lighting gives the user a luminous perception with beneficial effects during the treatment, also from the psychological-emotive point of view.

### DESCRIPTION OF THE DRAWINGS

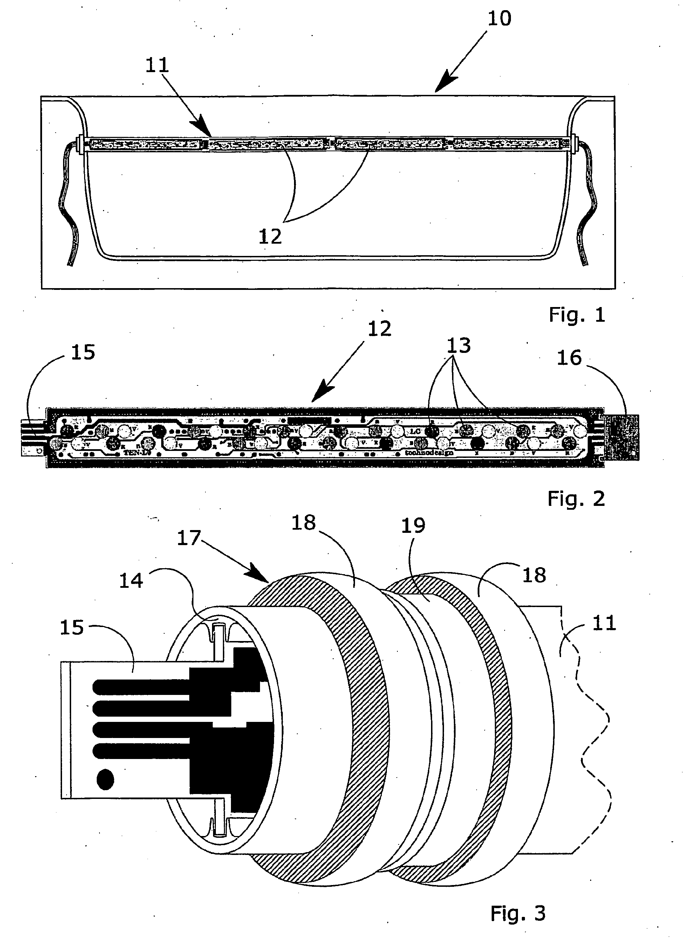

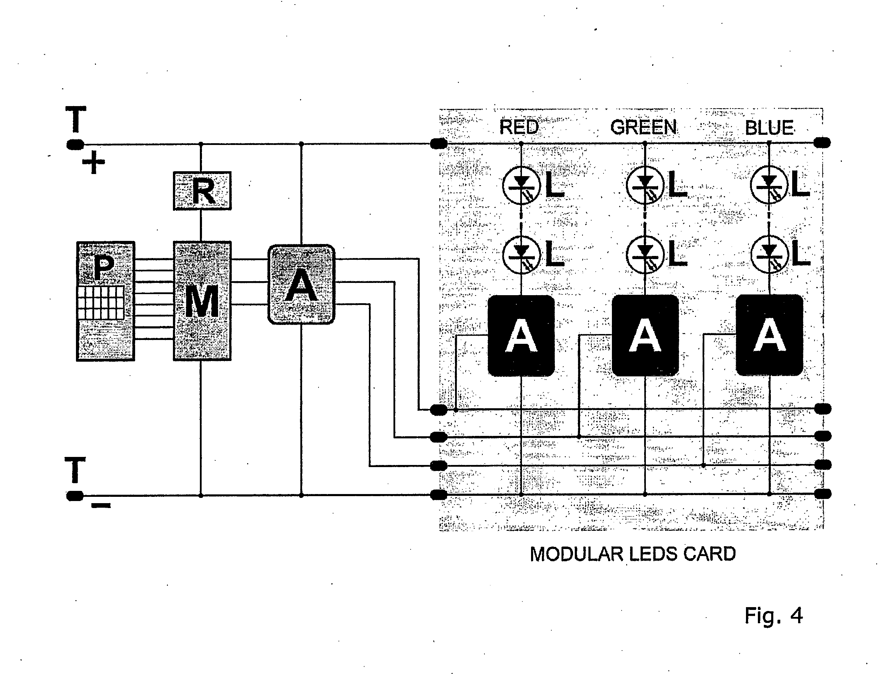

Other features and advantages of the invention will become evident on reading the following description of one embodiment of the invention, given as a non-binding example, with the help of the accompanying drawings in which:
■ figure 1 shows a transparency side view of a tub equipped with lighting means contained inside transparent tubular elements;
■ figure 2 is a schematic view of one of the modular lighting elements which can be joined together;
■ figure 3 represents a schematic view of a detail of one of the ends of the transparent tubes containing a lighting card;
■ figure 4 shows the diagram of a modular card fitted with variable chromatic LEDs and the relative microprocessor control system.

### DESCRIPTION OF ONE EMBODIMENT

In the figures, the reference number 10 indicates in general a tub for beauty, dermatological or other types of treatments, the tub preferably being provided with a wrapping sheet with a high degree of heat conduction, in the case in question a sheet fixed to the body of the tub for physical well-being and beauty treatment.

The main feature of the invention is the presence of a lighting system which is designed to generate diffused lighting with variable chromatics inside the tub to give the user psychological-emotional benefits during the treatment.

As can be seen in figures 1 and 3 the lighting system uses particular components, the main elements being:

One or more transparent tubular elements 11 made from polycarbonate, fibre glass reinforced plastic, glass, Plexiglas or other appropriate materials, which contain a plurality of electronic cards 12 each equipped with several luminous elements or LEDs, indicated with 13 in figure 2, with variable chromatics.

This system makes it possible to obtain lighting whose main feature is that it is distributed in a perfectly uniform way while ensuring maximum waterproofing of the system in terms of water leakage.

The electronic cards are in fact inserted inside tubular elements 11 by means of appropriately shaped tracks 14 which hold the cards in place and allow them to be inserted one after the other.

Quick-coupling means are used to connect the cards to each other, and can be the bayonet type or simple male-female couplings. The male-female couplings 15 and 16 are positioned at the ends of the cards, so that it is sufficient to press them together to achieve the electrical connection of the couplings.

The transparent tubular elements 11 are fixed permanently inside the tub. Holes are made in the walls of the tub (2 holes for each tube containing hundreds of LEDs) which allow the tubes to be inserted horizontally.

The ends of the tubular elements protrude on the outside of the tub and to ensure their fixing and sealing against any water leakage special seals 17 are used, consisting of substantially annular elements with raised edges 18 and a central groove 19.

When the tubular element is fitted, the hole made in the wall of the tub surrounds the central groove 19, while the transparent tubular element 11 remains inserted in the seal 17, forming a perfectly waterproof junction.

The ends of the tubular elements 11 are fitted with particular electrical couplings to which cables are attached that are connected to an electronic control unit or to a PLC or other appropriate control unit.

As already mentioned, each card can consist of a printed circuit comprising groups of LEDs. For example, there can be twelve groups of three different coloured LEDs, indicatively red, green and blue.

Figure 4 shows the wiring diagram of a modular element that makes up each group of three LEDs which, in the case in question, are red, green and blue.

For the lighting system to function, it is sufficient to programme the control unit to modulate the luminous intensity and the different shades according to a practically infinite number of combinations, with notable benefits for the user.

If the light sources are combined with the transparent flotation sheet, when a user lies on the sheet its base is deformed, wrapping itself around the user and thus ensuring good adhesion to the user's skin, while the light sources emit their colours according to the programmed intensities.

This condition favours heat exchange with the warm liquid contained in the tub and the simultaneous light emissions make it possible, thanks to the transparency of the sheet, to achieve psychological-perceptive benefits with all the consequent advantages for the result of the treatment session.

Advantageously, the luminous elements can also be used in sensory deprivation tubs or in tubs in which treatment is preferably carried out with the person motionless.

The invention is described above with reference to a preferred embodiment.

It is nevertheless clear that the invention is susceptible to numerous variations within the framework of technical equivalents as defined in the appended claims.

## Claims

1. A lighting system for tubs designed for therapeutic and/or beauty and/or dermatological treatments or designed to provide well-being with relaxation techniques, comprising a plurality of electronic cards each comprising lighting elements (10) with variable chromatics, **characterised in that** said lighting system comprises rigid tubes (11) made from transparent material, equipped with means (17) suitable for fixing them inside the body of these tubs and **in that** said rigid tubes contain said lighting elements (13).

2. A lighting system for tubs according to the foregoing claim, **characterised in that** the electronic cards (12) consist of printed circuits whose lighting elements consist of LEDs (13) of different colours.

3. A lighting system for tubs according to either of the foregoing claims, **characterised in that** the electronic cards (12) can be positioned alongside each other inside the transparent tubular elements, using rapid connection terminal boards which make them quick and easy to interchange.

4. A lighting system for tubs according to any of the foregoing claims, **characterised in that** the electronic cards (12) are inserted in the tubular elements (11) by means of appropriately shaped tracks (14) which hold the cards in place and allow them to be inserted one after the other.

5. A lighting system for tubs according to any of the foregoing claims, **characterised in that** to connect the electronic cards (12) together, rapid bayonet type coupling means are used or simple male-female couplings (15, 16) positioned at the ends of the cards so that it is sufficient to push one card against the next to achieve the electrical connection of the various couplings.

6. A lighting system for tubs according to any of the foregoing claims, **characterised in that** the ends of the tubular elements protrude outside the tub and to ensure their fixing and sealing against possible water leakage special seals (17) are used, consisting of substantially annular elements with raised edges (18) and a central groove (19).

7. A lighting system for tubs according to any of the foregoing claims, **characterised in that** the ends of the tubular elements (11) are fitted with particular electrical couplings to which cables are attached leading to an electronic control unit or to a PLC or other type of control unit.

## Patentansprüche

1. Beleuchtungssystem für Wannen, ausgebildet für therapeutische und/oder Schönheits- und/oder dermatologische Behandlungen oder ausgebildet, um Wohlbefinden mittels Entspannungstechniken bereit zu stellen, mit einer Mehrzahl von elektronischen Karten, von denen jede Beleuchtungselemente (10) aufweist, die variable Farben aufweisen, **dadurch gekennzeichnet, dass** das Beleuchtungssystem starre Röhren (11) umfasst, die aus einem transparenten Material gefertigt sind, die mit Mitteln (17) ausgestattet sind, die es ermöglichen, dass sie innerhalb des Körpers der Wannen fixiert werden und dass die starren Röhren die Beleuchtungselemente (13) aufnehmen.

2. Beleuchtungssystem für Wannen nach dem vorhergehenden Anspruch, **dadurch gekennzeichnet, dass** die elektronischen Karten (12) aus gedruckten Schaltkreisen bestehen, deren Beleuchtungselemente aus LED's (13) unterschiedlicher Farbe bestehen.

3. Beleuchtungssystem für Wannen nach irgendeinem der vorhergehenden Ansprüche, **dadurch gekennzeichnet, dass** die elektronischen Karten (12) im Inneren der transparenten rohrförmigen Elemente nacheinander positionierbar sind, unter Verwendung von Schnellverbindungsanschlussplatinen, so dass diese einfach und schnell ausgewechselt werden können.

4. Beleuchtungssystem für Wannen nach irgendeinem der vorhergehenden Ansprüche, **dadurch gekennzeichnet, dass** die elektronischen Karten (12) in die rohrförmigen Elemente eingesteckt werden, und zwar mittels geeignet geformter Führungen (14), die die Karten positioniert halten und die es erlauben, dass die Karten eine nach der anderen eingeführt werden.

5. Beleuchtungssystem für Wannen nach irgendeinem der vorhergehenden Ansprüche, **dadurch gekennzeichnet, dass** zur Verbindung der elektronischen Karten (12) untereinander bajonettartige Schnellverbindungsmittel oder einfache männliehe/weibliche Kopplungsmittel (15, 16), die an den Enden der Karten angeordnet sind, eingesetzt werden, so dass es ausreichend ist, eine Karten einzudrücken, gegen die nächste Karte, um eine elektrische Verbindung dieser unterschiedlichen Kopplungsmittel zu erreichen.

6. Beleuchtungssystem für Wannen nach irgendeinem der vorhergehenden Ansprüche, **dadurch gekennzeichnet, dass** die Enden der rohrförmigen Elemente über die Wanne nach Außen vorstehen, und dass deren Fixierung und Abdichtung gegen eine mögliche Wasser-Leckage sicher gestellt ist, nämlich durch spezielle Dichtungen (17), die benutzt werden, und die im wesentlichen aus ringförmigen Elementen bestehen, die erhabene Kanten (18) und eine zentrale Nut (19) aufweisen.

7. Beleuchtungssystem für Wannen nach irgendeinem der vorhergehenden Ansprüche, **dadurch gekennzeichnet, dass** die Enden der rohrförmigen Elemente (11) mit besonderen elektrischen Kopplungsmitteln ausgestattet sind, an denen Kabel angebracht sind, die zu einer elektronischen Kontrolleinheit oder einer PLC (programmierbare Logiksteuerungseinheit) oder einer anderen Art von Steuereinheit führen.

## Revendications

1. Système d'éclairage pour baignoires destiné à des traitements thérapeutiques et/ou cosmétiques et/ou dermatologiques ou destiné au bien-être avec des techniques de relaxation, ledit système comprenant une pluralité de cartes électroniques comprenant chacune des éléments d'éclairage (10) de divers chromatismes, **caractérisé en ce que** ledit système d'éclairage comprend des tubes rigides (11) en matière transparente et équipés de moyens (17) appropriés pour fixer lesdits tubes à l'intérieur du corps de ces baignoires et **en ce que** lesdits tubes rigides contiennent lesdits éléments d'éclairage (13).

2. Système d'éclairage pour baignoires selon la revendication précédente, **caractérisé en ce que** les cartes électroniques (12) consistent en des circuits imprimés dont les éléments d'éclairage consistent en des LED (13) de différentes couleurs.

3. Système d'éclairage pour baignoires selon l'une ou l'autre des revendications précédentes, **caractérisé en ce que** les cartes électroniques (12) peuvent être placées l'une à côté de l'autre à l'intérieur des éléments tubulaires transparents, en utilisant des cartes de bornes de connexion rapide qui permettent de les échanger facilement et rapidement.

4. Système d'éclairage pour baignoires selon l'une quelconque des revendications précédentes, **caractérisé en ce que** les cartes électroniques (12) sont insérées dans les éléments tubulaires (11) au moyen de pistes (14) de forme appropriée qui maintiennent les cartes en place et permettent de les insérer l'une après l'autre.

5. Système d'éclairage pour baignoires selon l'une quelconque des revendications précédentes, **caractérisé en ce que**, pour connecter les cartes électroniques (12) ensemble, on utilise des moyens de couplage rapide de type à baïonnette ou de simples raccords mâle/femelle (15, 16) placés aux extrémités des cartes de sorte qu'il suffit de pousser une carte contre la suivante pour réaliser la connexion électrique des divers raccords.

6. Système d'éclairage pour baignoires selon l'une quelconque des revendications précédentes, **caractérisé en ce que** les extrémités des éléments tubulaires font saillie à l'extérieur de la baignoire et assurent leur fixation et leur étanchéité vis-à-vis de fuite d'eau possible, des garnitures d'étanchéité spéciales (17) étant utilisées, lesquelles consistent en des éléments sensiblement annulaires présentant des rebords relevés (18) et une gorge centrale (19).

7. Système d'éclairage pour baignoires selon l'une quelconque des revendications précédentes, **caractérisé en ce que** les extrémités des éléments tubulaires (11) sont équipées de raccords électriques particuliers auxquels des câbles sont attachés, lesquels mènent à une unité de commande électronique ou à un PLC ou un autre type d'unité de commande.
